# EUROPEAN PATENT APPLICATION

(11) **EP 2 805 769 A1**
(43) Date of publication of application: **26.11.2014**
(21) Application number: 13169181.8
(22) Date of filing: 24.05.2013
(51) Int. Cl.: B01L 3/00, C12Q 1/68

(54) **Methods for nano-scale single cell analysis**

(71) Applicant: European Molecular Biology Laboratory, 69117 Heidelberg (DE)
(72) Inventor: Merten, Christoph A., 69115 Heidelberg (DE)
(74) Representative: Zwicker, Jörk

(57) **Abstract**

The present invention relates to methods for generating microcompartments comprising single cells, identifiers allowing to track the contents of each microcompartment and reagents for carrying out an analysis of the cell or its components. It also relates to high-throughput methods for analysing a plurality of single cells in parallel, including methods for single cell genomics, transcriptomics or methylation analysis.

## Description

The present invention relates to methods for generating microcompartments comprising single cells, identifiers allowing to track the contents of each microcompartment and reagents for carrying out an analysis of the cell or its components. It also relates to high-throughput methods for analysing a plurality of single cells in parallel, including methods for single cell genomics, transcriptomics or methylation analysis.

### BACKGROUND OF THE INVENTION

Cells have been assayed at the population level for more than a century. While this allows determining averaged properties, e.g. the average response of cells to a particular drug or the reference genome sequence of a particular species, many very important details remain undiscovered. In fact, assays with cell populations might even indicate a genotype or phenotype which, in reality does not exist (Toriello NM, et al. (2008) Integrated microfluidic bioprocessor for single-cell gene expression analysis. Proceedings of the National Academy of Sciences 105(51):20173-20178). For example, cell populations can consist of two clearly distinct cell types, one showing high expression of a particular gene and another one hardly expressing it at all. In an averaged readout, a non-existing mean phenotype is detected and the fact that the population comprises more than one cell type remains completely undiscovered. This is particularly bad in research fields dealing with very heterogeneous cells such as cancer, stem cells, developmental biology and metagenomics. While single cell transcriptomics could, for example, enable in-depths understanding of tumorigenesis (and potentially provide new therapeutic avenues), current approaches set very strict limits to novel discoveries.

Single cell genomics and transcriptomics are of major importance for the understanding of heterogeneous cell populations; e.g. in cancer, stem cell research or in developmental biology. The main challenge is to obtain significant read coverage in next generation sequencing (NGS) systems to fully assemble the genomes of individual cells and to detect subtle changes in the expression levels of particular (potentially low abundant) gene transcripts without significant bias. Furthermore, manual processing of individual cells is very time consuming and requires highly skilled personnel, for which reason an automated solution is urgently required.

Over the last couple of years, several academic and commercial platforms have been developed allowing to quantify the expression of a few (typically about 1-96) genes in individual cells (Sanchez-Freire V, Ebert AD, Kalisky T, Quake SR, & Wu JC (2012) Microfluidic single-cell real-time PCR for comparative analysis of gene expression patterns. Nature Protocols 7(5):829-838; Buganim Y, et al. (2012) Single-Cell Expression Analyses during Cellular Reprogramming Reveal an Early Stochastic and a Late Hierarchic Phase. Cell 150(6):1209-1222). These systems are based on qPCR and hence do not facilitate the analysis of global gene expression. Importantly, this does not only mean that less genes are analysed, but it implies that the genes to be studied have to be selected in the first place (e.g. based on prior knowledge) - again setting very strict limits for novel discoveries. Recently, global sequencing of DNA/RNA from manually manipulated single cells has been demonstrated by several groups (Tang FC, et al. (2010) RNA-Seq analysis to capture the transcriptome landscape of a single cell. Nature Protocols 5(3):516-535; Goetz JJ & Trimarchi JM (2012) Transcriptome sequencing of single cells with Smart-Seq. Nature Biotechnology 30(8):763-765; Lasken RS (2012) Genomic sequencing of uncultured microorganisms from single cells. Nature Reviews Microbiology 10(9):631-640). However the throughput and accuracy of these experiments is still unsatisfactory: No more than a very few cells can be analysed, and the obtained data neither allows efficient assembly of the full genome (in case DNA is used as starting material), nor quantitative analysis of low abundant gene transcripts. The latter is mainly due to biases in the sequence coverage introduced by inefficient reverse transcription or amplification artefacts.

The present inventor has overcome these limitations by applying micro fluidic technology on a nanolitre scale. Droplet-based microfluidic systems allow the compartmentalization of biological samples at very high frequencies. Up to several thousand samples per second can be processed. The resulting aqueous microcompartments (pico - nanoliter volumes) can be used as independent reaction vessels for a variety of applications, including (bio)chemical assays (Schaerli and Hollfelder, The potential of microfluidic water-in-oil droplets in experimental biology. Mol Biosyst (2009) vol. 5 (12) pp. 1392-404). When performing droplet fusion, droplet-based microfluidic systems also allow mixing individual samples at very high rates (up to kilohertz frequencies), thus generating samples of new/modified composition on-chip (Mazutis et al., A fast and efficient microfluidic system for highly selective one-to-one droplet fusion. Lab Chip (2009) vol. 9 (18) pp. 2665-2672).

Due to the tiny assay volumes (nanoliter range) the inventor can achieve, high concentrations (more than 70-fold higher compared to conventional approaches) of nucleic acids from individual cells. This massively improves the efficiency of the critical steps in the sequencing workflow and thus minimizes biases and artefacts. Furthermore, the technology allows a high level of automation, which not only increases throughput, but as well enhances reproducibility. Compared to a commercial RNAseq platform introduced just recently (c1™ Single-Cell Auto Prep System: http://www.fluidigm.com/clsystem.html), the system of the invention offers multiple advantages: While the commercial platform requires a large number of cells as starting material (∼2000) for filling just 96 single-cell traps, the present system is based on droplets into which cells can be encapsulated at almost 100% efficiency. Hence the approach of the inventor is much better suited for working with very rare cells (e.g. circulating tumour cells) or samples for which transcriptomic data of each individual cell is required (e.g. developing embryos). In addition, the system described here enables drastically increased throughput (e.g. up to 720 single-cell samples can be generated per hour), facilitates barcoding of each individual sample (to reduce sequencing costs by multiplexing) and is compatible with upstream FACS sorting. This way, specific cells of interest can be isolated using well established conventional technology and applied to RNAseq. Taken together, the technology of the inventor pushes the limits in several research fields (e.g microfluidic technology, biomedical applications, basic biology) and, due to its generic character, should enable the discovery of novel cell types, pathways and therapeutic approaches.

### DESCRIPTION OF THE FIGURES

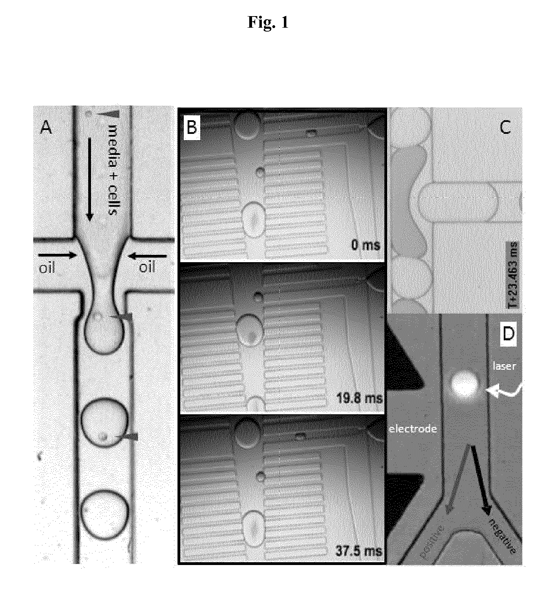

**Figure 1****: Droplet-based microfluidic modules. A)** Encapsulation of individual cells using a flow focussing droplet maker. Black arrows indicate the flow of the two immiscible phases; triangles highlight single mammalian cells. **B)** Time series of pillar-induced droplet fusion: Little substrate droplets (brown; flowing from right to left) coalesce with the initially formed large droplets (clear, flowing from top to bottom) and thus allow the addition of further compounds. **C)** Splitting of droplets (flowing from right to left towards the T-junction, where they split) **D)** Droplet sorting: Based on the quantitative fluorescence signal of individual droplets, embedded electrodes (black structures) are switched on and pull droplets of interest into the collection channel (left), while in absence of the electric field the droplets end up in the waste (right channel). Figures C and D are taken from Vyawahare, Griffiths & Merten, Chem Biol. 2010 vol. 17(10), pp. 1052-65.

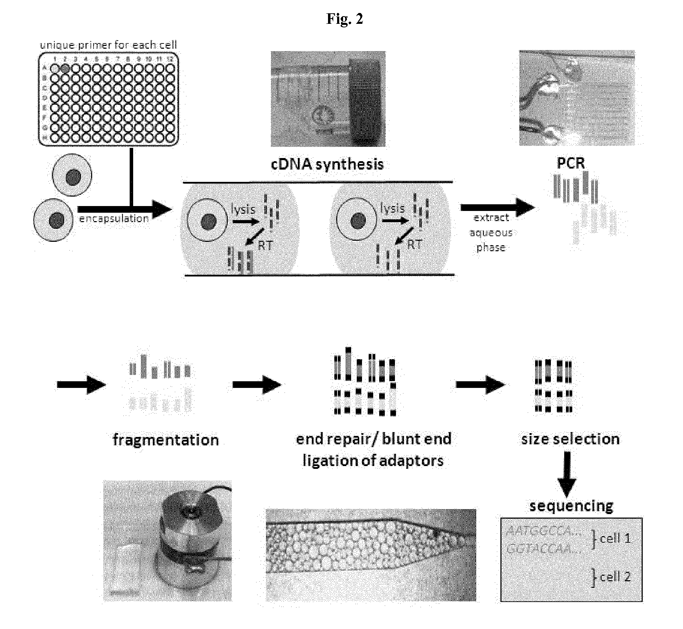

**Figure 2****: Workflow of microfluidic single cell transcriptomics.** Single cells are encapsulated into 70 nL plugs together with RT primers harboring a unique barcode for each sample (indicated by different scales of grey). This step is carried out by a custom-built autosampler platform loading the cells and primers from microtiter plates. Subsequently, the first strand synthesis is carried out in the microcompartments, before the aqueous samples are pooled for further processing (note that the barcodes still allow to determine which cDNAs were generated from the same cell). Second strand synthesis and pre-amplification of the newly synthesized cDNA are perfomed on-chip in continuous (single-phase) flow systems, or, optionally in emulsions (ePCR). In a further step the sample is sonicated, resulting in sheared DNA with a size of about 200bp (as required for NGS machines). Then, the DNA is end-repaired, adaptors are ligated and size selection is performed using solid-phase modifications on Ampure XP beads (in microfluidic bead columns). Finally the sample is eluted and sequenced on commercial NGS platforms. By pooling the samples directly after the first strand synthesis sample loss (e.g. by adhesion to the channel walls) can be minimized. Images show the existing microfluidic modules for performing the individual steps (image of the sonicator adapted from Tseng Q, Lomonosov AM, Furlong EE, Merten CA. Fragmentation of DNA in a sub-microliter microfluidic sonication device. Lab Chip. 2012 vol 12(22), pp. 4677-82).

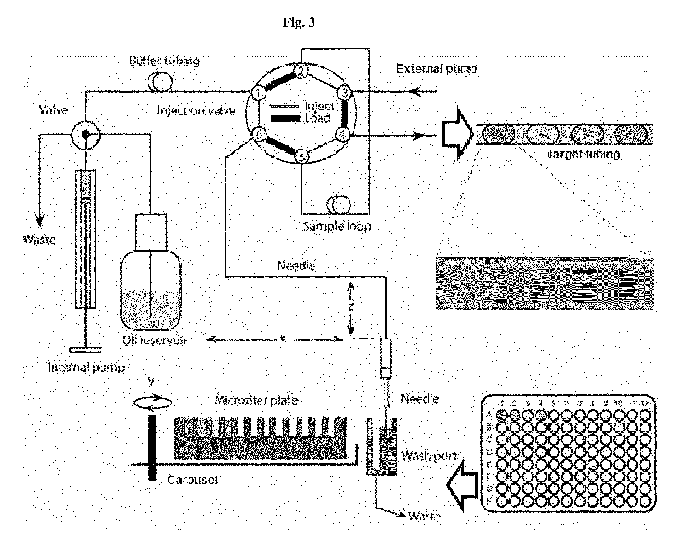

**Figure 3****: Setup for the loading of samples from microtitre plates into individual drops/plugs.** The central injection valve of an autosampler controls the fluidic pathways. In the load position (indicated by thick lines) aqueous samples are aspirated from microtiter plates (bottom center: side view; bottom right: top view) into the sample loop by an internal pump. In parallel, the target tubing is filled with oil coming from the external pump. When the valve switches into the inject position (thin lines), the sample loop is purged with the oil from the external pump. In parallel, the buffer tubing and the needle are washed with oil coming from the internal pump (fed by an internal reservoir). Image adapted from Clausell-Tormos, J., Griffiths, A.D. & Merten, C.A. An automated two-phase microfluidic system for kinetic analyses and the screening of compound libraries. Lab Chip. 2010 10(10) pp. 1302-7. Inset: Single cells preloaded in the wells (e.g. during a prior FACS sort) can be encapsulated, too. Using additional fluorinated oil in the wells facilitates aspirating from the sample, even the entire sample, without the generation of air bubbles.

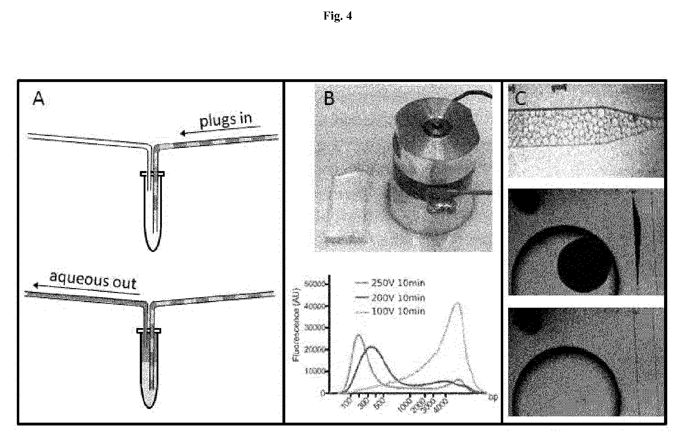

**Figure 4****: Microfluidic modules. A)** Extraction of aqueous samples from two-phase (plug) systems. The plugs are flushed into a sealed tube (top) where, based on the different densities (fluorinated oil has an approximately 2-fold higher density compared to water), the two phases separate. In presence of an emulsion breaker (e.g. perfluoro-octanol) this setup even works for surfactant-stabilized emulsions. **B)** Microfluidic sonicator enabling the shearing of genomic DNA. Top: Setup of the device: A Langevin type piezo transducer is glued to the glass surface and generates strong acoustic fields in a neighbouring PDMS chip Bottom: Fluorescent line scan of the sheared DNA after sonication of 500 ng cross-linked chromatin from Drosophila melanogaster embryos. Different voltages and pulse durations are indicated by different colors. Image adapted from Tseng Q, Lomonosov AM, Furlong EE, Merten CA. Fragmentation of DNA in a sub-microliter microfluidic sonication device. Lab Chip. 2012 vol 12(22), pp. 4677-82. C) Microfluidic modules for performing biochemical manipulations. Top: Microfluidic bead column allowing purification and washing of bound DNA/RNA. Center and bottom: Embedded magnets (dark round structure) enable the trapping of functionalized magnetic beads in an adjacent microfluidic channel. Upon removal of the magnet the beads can be released for further processing.

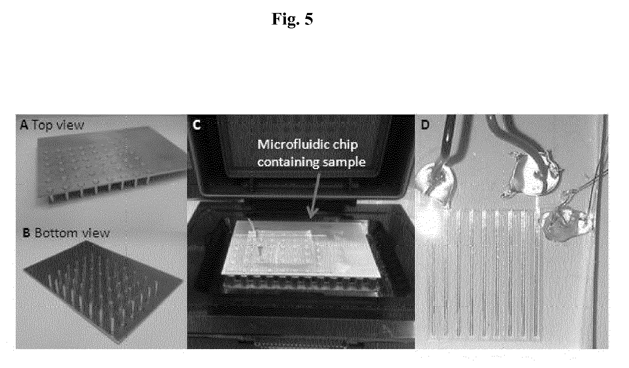

**Figure 5****: PCR in microfluidic channel networks. A) & B)** Custom-built aluminium plate for homogeneously distributing heat from a conventional thermo cycler. The pins are immersed in tube holders filled with oil. **C)** Microfluidic chips can be mounted on top of the flat surface, hence allowing to thermo cycle samples within the channels. **D)** Heating element directly integrated into a microfluidic channel network. The temperature can be adjusted by the applied voltage and measured using an embedded thermistor.

### SUMMARY OF THE INVENTION

Generally, the present invention relates to the field of single cell analysis in small volumes.

In a first aspect, the present invention relates to a method for generating a plurality of aqueous microcompartments having a volume of up to 1 micro litre, each comprising:
- a single cell, and
- at least one identifier comprising a molecular barcode being unique for each microcompartment.

In a second aspect, the present invention relates to an aqueous microcompartment having a volume of up to 1 microlitre, comprising:
- a single cell, and
- at least one identifier comprising a molecular barcode being unique for each microcompartment.

In a third aspect, the present invention relates to a sample generated by merging at least two aqueous microcompartments according to the second aspect.

In a fourth aspect, the present invention relates to a method for modifying, amplifying and/or analysing the DNA or RNA comprised in a single cell, wherein the cell is comprised in a microcompartment according to the second aspect for at least some of the method steps.

This summary of the invention does not necessarily describe all features of the invention.

### DETAILED DESCRIPTION OF THE INVENTION

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland).

Several documents are cited throughout the text of this specification. Each of the documents cited herein (including all patents, patent applications, scientific publications, manufacturer's specifications, instructions, GenBank Accession Number sequence submissions etc.), whether supra or infra, is hereby incorporated by reference in its entirety. Nothing herein is to be construed as an admission that the invention is not entitled to antedate such disclosure by virtue of prior invention.

In the following, the elements of the present invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

Throughout this specification and the claims which follow, unless the context requires otherwise, the word "comprise", and variations such as "comprises" and "comprising", are to be understood to imply the inclusion of a stated integer or step or group of integers or steps but not the exclusion of any other integer or step or group of integer or step.

As used in this specification and the appended claims, the singular forms "a", "an", and "the" include plural referents, unless the content clearly dictates otherwise.

The present inventor has devised a fully integrated microfluidic platform, capable of preparing single cell samples for cell analysis, e.g. RNA or DNA sequencing at high throughput (including all steps of the sequencing library preparation). Making use of nanoliter sample volumes (allowing to obtain more than 70-fold higher concentrations of DNA/RNA from a single cell as compared to current bench top protocols) the system drastically increases the efficiency of reverse transcription and amplification steps, hence reducing biases observed in conventional systems to an absolute minimum. Furthermore, by using primers with a unique barcode for each individual cell, multiplexing becomes feasible and minimizes the sequencing costs. In fact, this approach enables to pool the samples, which also reduces sample loss, e.g. by adhesion of DNA molecules to the walls of the microfluidic channels. Taken together, the system of the invention allows to drastically increase the throughput (>>100 samples in a single experiment), minimize the sequencing costs and diminish sequencing bias as seen for conventional systems - all in a fully automated way.

In a first aspect, the present invention relates to a method for generating a plurality of aqueous microcompartments having a volume of up to 1 micro litre, each comprising:
- a single cell, and
- at least one identifier comprising a molecular barcode being unique for each microcompartment.

The term "plurality" means "more than one". In preferred embodiments of the methods of the first aspect, it means more than 10, more than 20, more than 30, more than 40, more than 50, more than 100, more than 250, more than 500 or more than 1000.

The term "aqueous microcompartments" refers to compartments of a certain size that encapsulate an aequeous liquid. The size or volume of the aqueous microcompartments of the invention is less than 1 microlitre. Preferably, it is less than 500 nanolitres, less than 250, less than 150, less than 100 or less than 50 nl. In a preferred embodiment, it is between 0.1 and 150 nl, preferably between 1 and 125 nl, between 10 and 100 nl or between 30 and 80 nl. In one preferred embodiment, it is about 70 nl.

The function of the compartment is to enable co-localisation of a cell and an identifier as well as reagents for carrying our various reactions on the cell or its components. This is achieved by the ability of the microcompartment to substantially restrict diffusion of its contents to other microcompartments. Any reactions in the microcompartments are therefore restricted to being exercised on the cell or its components within the confines of their microcompartment.

A wide variety of compartmentalisation or microencapsulation procedures are available (Benita, S., Ed. (1996). Microencapsulation: methods and industrial applications. Drugs and pharmaceutical sciences. Edited by Swarbrick, J. New York: Marcel Dekker) and may be used to create the compartments used in accordance with the present invention. Indeed, more than 200 microencapsulation or compartmentalisation methods have been identified in the literature (Finch, C. A. (1993) Encapsulation and controlled release. Spec. Publ.-R. Soc. Chem. 138, 35). These include membrane enveloped aqueous vesicles such as lipid vesicles (liposomes) (New, R. R. C., Ed. (1990). Liposomes: a practical approach. The practical appraoch series. Edited by Rickwood, D. & Hames, B. D. Oxford: Oxford University Press) and non-ionic surfactant vesicles (van Hal, D. A., Bouwstra, J. A. & Junginger, H. E. (1996). Nonionic surfactant vesicles containing estradiol for topical application. In Microencapsulation: methods and industrial applications (Benita, S., ed.), pp. 329-347. Marcel Dekker, New York.). Preferably, the microcompartments of the present invention are formed from emulsions; heterogeneous systems of two immiscible liquid phases with one of the phases dispersed in the other as droplets of microscopic size (Becher, P. (1957) Emulsions: theory and practice. Reinhold, New York; Sherman, P. (1968) Emulsion science. Academic Press, London; Lissant, K.J., ed Emulsions and emulsion technology. Surfactant Science New York: Marcel Dekker, 1974; Lissant, K.J., ed. Emulsions and emulsion technology. Surfactant Science New York: Marcel Dekker, 1984). Emulsions may be produced from any suitable combination of immiscible liquids. Preferably the emulsion of the present invention has water (containing a cells, identifiers and other components) as the phase present in the form of droplets and a hydrophobic, immiscible liquid (preferably an oil) as the surrounding matrix in which these droplets are suspended. Such emulsions are termed 'water-in-oil'. This has the advantage that the aqueous phase is compartmentalised in discrete droplets. The external phase, preferably being a hydrophobic oil, generally is inert. The emulsion may be stabilised by addition of one or more surface-active agents (surfactants). These surfactants act at the water/oil interface to prevent (or at least delay) separation of the phases. Many oils and many emulsifiers can be used for the generation of water-in-oil emulsions; a recent compilation listed over 16,000 surfactants, many of which are used as emulsifying agents (Ash, M. and Ash, I. (1993) Handbook of industrial surfactants. Gower, Aldershot). Suitable oils are listed below.

Preferably, the aqueous microcompartments are created, handled and/or controlled in a microfluidic system. This technology is based on the manipulation of continuous liquid flow through microfabricated channels. Actuation of liquid flow is implemented either by external pressure sources, external mechanical pumps, integrated mechanical micropumps, or by combinations of capillary forces and electrokinetic mechanisms. These closed-channel systems are inherently difficult to integrate and scale, because the parameters that govern flow field vary along the flow path making the fluid flow at any one location dependent on the properties of the entire system. Process monitoring capabilities in continuous-flow systems can be achieved with highly sensitive microfluidic flow sensors based on MEMS technology which offer resolutions down to the nano liter range.

Microfluidic devices typically consist of networks of channels of approximately ten to a few hundred micrometers in diameter into which small quantities of reagents can be injected in a specific sequence, mixed and incubated for a specified time. Assays can be highly parallelized by generating independent compartments using valves (pinching off specific regions of the channels) or two-phase microfluidics, in which aqueous droplets surrounded by an immiscible oil phase serve as closed vessels. These approaches enable drastically reduced assay volumes (pico - nanoliters) and strongly improved throughput. For example, droplets can be generated at rates of more than 1,000 per second. Furthermore, microfluidic modules for the splitting, fusion and sorting of droplets at similar rates have been developed, thus providing a repertoire of manipulations mimicking classical bench top procedures.

Thus, the microcompartment of the invention can also be termed "microfluidic droplet" or "microfluidic plug", e.g. of a water-in-oil emulsion (Schaerli and Hollfelder, The potential of microfluidic water-in-oil droplets in experimental biology. Mol Biosyst (2009) vol. 5 (12) pp. 1392-404; Clausell-Tormos et al., An automated two-phase microfluidic system for kinetic analyses and the screening of compound libraries. Lab Chip. (2010) vol. 10 (10) pp. 1302-7.).

The cell can be any prokaryotic or eukaryotic cell. Preferably, it is a prokaryotic cell, yeast cell, plant cell or animal cell. Animal cells include insect, nematode, fish and mammalian cells. More preferably it is mammalian cell, e.g. a mouse, rat, monkey or human cell. For example, it can be a random cell of a heterogenous cell population or it can be a specifically selected cell, selected, e.g., by FACS. Preferred cells are developing cells, stem cells or cancer cells.

The term "identifier" refers to any molecule which allows the identification or tracking of the aqueous microcompartment it is or was comprised in. Generally, it comprises a unique composition or chemical moiety, allowing unambiguous identification. For example, an identifier can be a sequencing identifier, e.g. an oligonucleotide, a spectroscopy identifier, preferably a mass spectroscopy identifier, preferably a peptide, or an optical identifier, preferably an oligonucleotide (Geiss et al., Direct multiplexed measurement of gene expression with color-coded probe pairs. Nature Biotechnology (2008) vol. 26 (3) pp. 317-25) or a quantum dot. Other examples are graphical or electronic identifiers. An identifier which is useful in the present invention preferably possesses one or more of the following attributes:
1) It is capable of being distinguished from all other identifiers.
2) The identifier is capable of being detected when present in low amounts, for example at 10 ⁻²² to 10 ⁻⁶ mol.
3) The identifier possesses a chemical handle through which it can be attached to a substrate (e.g. DNA or RNA of a cell), wherein the substrate can be a carrier.
4) The identifier is chemically stable towards all manipulations to which it is subjected and which are not meant to degrade it, in particular those that occur during steps of the methods of the present invention described herein.
5) The identifier does not significantly interfere with the manipulations performed on the substrate it can be attached to (unless intended to).

In a preferred embodiment, the identifier is a sequencing identifier. A sequencing identifier is any molecule which is characterised by a sequence of subunits, which can be identified by sequencing methods. Preferably, it is an oligonucleotide (e.g. RNA, DNA or PNA), which can be analysed, for example, by classical sequencing (e.g. Sanger sequencing) or next-generation sequencing (Metzker, Sequencing technologies - the next generation. Nat Rev Genet (2009) viol. 11(1) pp. 31-46).

The term "molecular barcode" refers to any characteristic of the above-described identifiers which allows distinguishing one identifier from another. Molecular barcodes are unique molecules associated with a particular specimen sample and they are transmitted with the specimen. To track a particular specimen in a sample containing many different specimens, one identifies the molecular barcodes using a preferably simple method applicable to all molecular barcodes used. The most preferably used barcode molecules are oligonucleotides due to simple unique design possibilities and cost-effective and established identification technologies such as sequencing or microarray hybridization.

Thus, preferably, the molecular barcode is a barcode sequence of a sequence identifier, preferably of an oligonucleotide.

In a preferred embodiment, the identifier is a sequence identifier, preferably an oligonucleotide, comprising a constant sequence and a barcode sequence, wherein the constant sequence is identical for all microcompartments and the barcode sequence is unique for each microcompartment.

Preferably, the sequence identifier oligonucleotide is an RNA, DNA or a PNA molecule. Peptide nucleic acids (PNAs) are compounds that in certain respects are similar to oligonucleotides and their analogs and thus may mimic DNA and RNA. In PNA, the deoxyribose backbone of oligonucleotides has been replaced by a pseudo-peptide backbone (Nielsen et al. 1991 Science 254, 1457-1500). Each subunit, or monomer, has a naturally occurring or non-naturally occurring nucleobase attached to this backbone. One such backbone is constructed of repeating units of N(2-aminoethyl) glycine linked through amide bonds. PNA hybridises with complementary nucleic acids through Watson and Crick base pairing and helix fold. The pseudo-peptide backbone provides superior hybridization properties (Egholm et al. Nature (1993) 365, 566-568), resistance to enzymatic degradation (Demidov et al. Biochem. Pharmacol. (1994) 48, 1310-1313) and access to a variety of chemical modifications (Nielsen and Haaima Chemical Society Reviews (1997) 73-78).

The length of the sequence identifier oligonucleotide preferably has a length of between 4 and 200, between 4 and 100, between 10 and 80, or between 30 and 70 nucleotides.

The term "constant sequence" refers to a part of the sequence identifier which is identical for all microcompartments. If one microcompartment comprises more than one identifier, it is preferred that the constant sequence of the at least two identifiers are different from each other, but that each one is identical to one constant sequence in a different microcompartment. Preferred examples for constant sequences are inverted repeats of transposable elements (e.g., for a transposition reaction) or sequences capable of hybridization to a DNA or RNA sequence comprised in the cell of the same microcompartment (e.g. for a reverse transcription reaction or a reaction for amplifying bisulfite treated DNA). Such sequences capable of hybridization to a DNA or RNA sequence can act as primer sequences, such as the sequences of poly(dT) primers which are capable of hybridising to the poly(A) tail ofmRNA (to determine the RNA of the single cell), or a 3' sequence (meaning at the 3' end of the sequence identifier oligonucleotide) specific for bisulfit converted genomic DNA, which hybridises upstream of a genomic DNA sequence comrising at least one CpG site. Inverted repeats of transposable elements preferably are 3' and 5' end sequences surrounding the barcode sequence. An example are the 19 bp Tn5 end sequences for Tn5 transposition (see Goryshin and Reznikoff, J Biol Chem. 1998 Mar 27;273(13):7367-74),

The term "barcode sequence" refers to a part of the sequence identifier which is unique for each microcompartment. Preferably, it is a nucleotide sequence comprising a sequence which differs in at least one nucleotide from the sequences of at least all other barcode sequences having the same constant sequence. It may or may not be the identical for all barcode sequences of sequence identifiers in the same microcompartment having different constant sequences. Also, it may or may not be identical to barcode sequences of sequence identifiers of other microcompartments having a different constant sequence. In a preferred embodiment, it is identical to all barcode sequences of sequence identifiers in the same microcompartment having different constant sequences and/or different from barcode sequences of sequence identifiers of other microcompartments. Different means that two barcode sequences differ in at least 1, at least 2, at least 3, at least 4, at least 5, at least 10 or at least 15 of 4, 5, 10 or 15 to 100 positions.

In embodiment A, the method of the first aspect comprises the steps of:
(i) providing an array of wells,
(ii) seeding cells comprised in an aqueous medium into at least two wells of the array of wells in such that only one cell is seeded into each well,
(iii) adding at least one identifier into at least two wells of the array of wells, such that each of the at least two wells is assigned a different barcode sequence, and
(iv) adding to at least two wells a liquid, which is immiscible with the aqueous medium comprising the cells of (ii) and has a higher density than the aqueous medium,
(v) in an alternating fashion, (a) aspirating the volume of up to 1 microlitre at the interphase after the cell has sedimented at the interphase from one well into a microchannel containing the immiscible liquid, and (b) filling the microchannel with the immiscible liquid behind the aspirated aequous volume,
wherein steps (ii), (iii) and (iv) can be carried out in any order or simultaneously.

An "array of wells" can be a plate of any shape containing a plurality of wells, e.g. 50 to 2000 wells, with a well diameter of about 0,5 to 50 mm. Preferred are microtiter plates according to the ANSI standard, e.g. 48-, 96-, 384- or 1536-well plates.

The term "seeding cells" refers to adding a single cell into each well. Seeding cells "such that only one cell is seeded into each well" can be achieved by any suitable means. For example, cells can be seeded into wells using FACS or picked individually from a heterogeneous sample using a micro-manipulator and then added to a well. This way, a specific cell can be assigned to a well. Also, a medium comprising cells can be diluted such that the volume added to each well has a statistical likelihood of containing exactly one or less than one cell. One or less than one cell preferably means >0 to 0.8, preferably 0.1 to 0.8, more preferably 0.1 to 0.6, and most preferably 0.2 to 0.4 cells on average.

The "immiscible liquid" is preferably a hydrophobic liquid, preferably an oil. It is preferred that its density is higher than that of the aqueous medium or higher than that of water. More preferably, its density is at least 0.99127 g/cm³ (the density of water at 37°C), preferably at least 1.000 g/cm³ (the highest density of water, i.e. the density at 4°C). Further preferred densities are at least 1.500 and at least 2.000 g/cm³, which improve phase separation.

The oil phase should allow the microcompartments to be stable against coalescence, have a viscosity that is close to that of water, and/or be inert with respect to the biological reagents contained in them. Several oils can be used. Low-viscosity silicone oils swell PDMS (Polydimethylsiloxane, called PDMS or dimethicone, is a polymer widely used for the manufacture of microfluidic chips; it is a mineral-organic polymer (a structure containing carbon and silicon) of the siloxane family), changing the cross-sectional dimensions of microfluidic channels and influencing the flow properties of microfluidic devices. This also depletes the carrier phase from the channels, limiting on-chip incubation time and interfering with droplet recovery. Silicone oils can also be used in microfluidic devices fabricated in glass, which are impermeable to these oils. High-viscosity silicone oils can be used in PDMS devices with minimal swelling at the expense of significantly increasing the pressures required to pump them through the microchannels. Hydrocarbon oils can also be obtained in a range of viscosities and have the benefit that there are a large number of commercially available surfactants for them that can stabilize aqueous-in-oil emulsions. However, they also swell PDMS and tend to exhibit poor retention of encapsulated organic reagents, which are often partially soluble in these oils. The preferred oils for use in the methods of the invention are fluorocarbon oils (or flourinated oils), because even low-viscosity versions of these oils do not swell PDMS. In addition, they tend to exhibit excellent retention of reagents in the drops and have high solubility for gases, allowing oxygen and carbon dioxide to passively diffuse in and out of the microcompartments, for unperturbed cellular respiration. This allows cells to survive in fluorocarbon oil emulsions for hours after encapsulation. A disadvantage of fluorocarbon oils, however, is that, due to their much lower prevalence compared with silicone and hydrocarbon oils, there are fewer commercially available surfactants for stabilizing aqueous-in-fluorocarbon emulsions. Surfactants are useful for reducing the surface tension of the oil-water interface and minimizing droplet coalescence. The choice of which surfactant to use is also important for limiting the transfer of reagents between microcompartments. A comprehensive review of surfactants for droplet-based micro fluidics is given in Baret (2012 Lab Chip 12, 422). The surfactants utilized in droplet-based microfluidics normally consist of a hydrophilic head group and hydrophobic tail. The amphiphilic character of these molecules allows them to assemble at the oil-water interface of the droplet, thereby lowering its interfacial tension and enhancing stability. The chemical properties of the head group of the surfactant impact the biocompatibility of the droplet interface. Surfactants with non-ionic head groups are preferred, as they minimize the adsorption of macromolecules such as proteins and DNA to the droplet interface, minimally impacting the methods of the invention. Suitable fluorosurfactants that can be readily synthesized in the lab are known in the art and described, e.g., in Clausell-Tormos J et al 2008 Chem. Biol. 15, 427-37 or Sadtler et al. 1996 Angew. Chem. Int. Edn Engl. 35, 1976-8. Additives to the aqueous phase can also enhance biocompatibility by increasing the retention of small molecules in the droplets and minimizing adsorption at the oil-water interface. Different oils can be mixed to optimize the properties of the emulsion for a particular application and methods for easily characterizing the properties of the combination that has been selected are known in the art (Kaltenbach et al. 2012 Lab Chip 12, 4185).

The term "aspirating" relates to taking up the volume by using a force generated by suction through any suitable device, e.g. a needle. As the cell sediments at the interphase, the aspirated volume comprises the cell. The skilled person will understand that the diameter of the well must not be too large, otherwise the aspirated volume may not comprise the cell. The wells of the array of wells described above are suitable for the aspirating step of the method of embodiment A of the first aspect of the invention.

Preferably, in step (v), the volume of up to 1 microlitre is aspirated from the aqueous side of the interphase.

In a preferred embodiment, the immiscible liquid is added the the well(s) in a volume sufficient to achieve an immiscible liquid column in the well, wherein the liquid column has a height in the range of 0.01 mm to a height below the top of the well, sparing sufficient room for the medium comprising the cell, immersing the aspirating means and other components to be added. Preferably, the immiscible liquid column in the well has a height of 0,1 to 10 mm, more preferably 0.5 to 0.8 mm and most preferably 1 to 4mm. Preferably, the immiscible liquid is added into the wells before addition of the cell. This facilitates aspirating from the sample, even the entire sample, without the generation of air bubbles

The term "microchannel" refers to a channel of a diameter of less than 1000 µm, preferably less than 900, 800, 700, 600, 500, 400, 300, 200, 150, 100, 50, 40, 30, 20, 10, 5 or 1 µm, preferably the diameter is between 50 nm and 500 µm, preferably between 100 nm and 100 µm and more preferably between 200 nm and 50 µm In a preferred embodiment, the microchannel is part of a microfluidics network. If the microchannel does not have a circular cross-section the cross section is preferably of the size of a circular channel having the diameters and preferred diameters indicated above.

In an embodiment B, the method of the first aspect comprises the steps of:
(i) injecting (a) an aqueous medium comprising cells and (b)sequentially at least one identifier per cell into a microchannel to create a laminar co-flow of cells and identifiers, wherein the sequential injection of the identifiers is controlled by at least one valve,,
(ii) generating cell droplets in the microchannel using microfluidic flow focusing from the laminar co-flow of step (i), wherein the concentration of the cells in the medium and the droplet size are adjusted such that each drop has a statistical likelihood of comprising less than one cell.

The term "laminar co-flow" refers to the flow of liquids in parallel layers, with no disruption between the layers, i.e. the opposite of turbulent flow. In laminar flow the motion of the particles of fluid is very orderly with all particles moving in straight lines parallel to the microchannel walls. In the case of flow through a straight microchannel with a circular cross-section, at a Reynolds number (ratio of inertial forces to viscous forces) below the value of approximately 2040, fluid flow is laminar.

In one embodiment, step (i) may comprise the injection of (c) further components to be comprised in the microcompartment. Such further components may be proteins, small molecules or salts.

The term "droplet" refers to a microcompartment as defined above, but with a volume that can be greater than 1 microlitre, e.g. up to 5 or up to 10 microlitres. The term "cell droplet" refers to a droplet which has a statistical likelihood of containing 1 or less than 1 cell (as defined above), or a droplet derived therefrom. Thus, the term "cell droplet" determines purpose and/or origin rather than the content of the droplet actually comprising a cell. A cell droplet does preferably not comprise identifiers. Alternatively, it may also be referred to as "first droplet". The term "identifier droplet" (or "second droplet") refers to a droplet comprising at least one identifier as defined above, but no cells.

Cell droplets and microcompartments not containing a cell (e.g. because they have been splitted or they do not contain a cell because of the likelihood of containing one is below 1) can be processed as those containing a cell. In this case, the methods of the invention relying on the presence of a cell will not yield any results and such negative samples are simply ignored. Alternatively, cell droplets and microcompartments containing a cell can be selected for further processing, e.g. by labeling the cell. The cell can be labeled before it is compartmentalized or after. A label can, for example, be added together with or in the same way as the identifier. Cell surface antibodies, e.g. coupled to a fluorescent moiety, are examples for suitable cell labels. In a preferred embodiment, sorting is achieved based on the fluorescence intensity of the droplets (Baret et al., Fluorescence-activated droplet sorting (FADS): efficient microfluidic cell sorting based on enzymatic activity. Lab Chip (2009) vol. 9 (13) pp. 1850-1858). Other cell sorting methods can be based on physical properties of the cells, such as size, deformability, electric, acoustic or optical properties.

"Microfluidic flow focusing" is a hydrodynamic means for the production of droplets. The principle consists of a continuous phase fluid (focusing fluid) flanking or surrounding the dispersed phase (focused fluid), so as to give rise to droplet break-off in the vicinity of an orifice through which both fluids are extruded. A flow focusing device comprises of a pressure chamber pressurized with a continuous focusing fluid supply. Inside, one or more focused fluids are injected through a capillary feed tube whose extremity opens up in front of a small orifice linking the pressure chamber with the exterior ambient. The focusing fluid stream moulds the fluid meniscus into a cusp giving rise to a steady micro or nano-jet exiting the chamber through the orifice; the jet size is much smaller than the exit orifice, thus precluding any contact (which may lead to unwanted deposition or reaction). Capillary instability breaks up the steady jet into homogeneous droplets or bubbles. See also Anna, S.L., Bontoux, N., and Stone, H.A. (2003). Formation of dispersions using "flow focusing" in microchannels. Appl. Phys. Lett. 82, 364-366.

The droplet size can be controlled by adjusting the flow rate and the drop frequency such that each drop has a statistical likelihood of comprising less than one cell.

In a preferred embodiment, in step (I) of embodiment B, the sequential injection of the identifiers is controlled either by one valve for all identifiers, fed with one identifier after the other, or by one valve per identifier, wherein one valve opens after the other and not more than one valve is open at any time. The sequential injection by one valve for all identifiers can be achieved, for example, by feeding the valve with an autosampler.

In a further preferred embodiment, at least two identifiers or at least 3, 4, 5, 6, 7, 8, 9, 10, 20, 50, 75 or at least 100 identifiers are injected into the microchannel to be combined with one single cell. These identifiers are preferably injected sequentially. In one embodiment, the sequential injection of the at least two identifiers is controlled (i) by one valve for all identifiers, fed with one identifier after the other, or (ii) by one valve per identifier, wherein one valve opens after the other and not more than one valve is open at any time. The sequential injection by one valve for all identifiers can be achieved, for example, by feeding the valve with an autosampler.

The at least two identifiers can be identifiers of the same type, i.e. identifiers comprising a 3' oligo(dT) sequence, a transposable element for an *in vitro* transposition reaction or a 3' sequence specific for bisulfit converted genomic DNA. Alternatively, they can be identifiers of different types, i.e. an identifiers comprising a 3' oligo(dT) sequence, a transposable element for an *in vitro* transposition reaction and/or a 3' sequence specific for bisulfit converted genomic DNA. Examples for such combinations of types of identifiers are:
- at least one identifier comprising a 3' oligo(dT) sequence and at least one identifier comprising a transposable element for an *in vitro* transposition reaction,
- at least one identifier comprising a 3' oligo(dT) sequence and at least one identifier comprising a 3' sequence specific for bisulfit converted genomic DNA,
- at least one identifier comprising a transposable element for an *in vitro* transposition reaction and at least one identifier comprising a 3' sequence specific for bisulfit converted genomic DNA,
- at least one identifier comprising a 3' oligo(dT) sequence, at least one identifier comprising a transposable element for an *in vitro* transposition reaction, and at least one identifier comprising a 3' sequence specific for bisulfit converted genomic DNA.

Preferably, in step (ii) of embodiment B, the cell droplets are generated such that each cell comprises the identifier(s) it co-flows with.

In an embodiment C, the method of the first aspect comprises the steps of:
(i) generating cell droplets in a first microchannel using microfluidic flow focusing from an aqueous medium comprising cells, wherein the concentration of the cells in the medium and the drop size are adjusted such that each drop has a statistical likelihood of comprising less than one cell,
(ii) generating oligonucleotide droplets in a second microchannel, preferably using microfluidic flow focusing, from a liquid comprising the at least one identifier, wherein each droplet comprises an identifier with a different barcode sequence,
(iii) fusing each cell droplet with an identifier droplet,
wherein steps (i) and (ii) can be carried out in any order or simultaneously.

"Fusing" two droplets results in one droplet comprising the contents of the two origin droplets. Thus, in the above embodiment, the droplet resulting from fusion comprises the contents of the cell droplet, i.e. a cell (with a likelihood of less than 1), and the at least one identifier. One-to-one fusion can be achieved, e.g., according to Mazutis et al. (A fast and efficient microfluidic system for highly selective one-to-one droplet fusion. Lab Chip (2009) vol. 9 (18) pp. 2665-2672).

In an embodiment D, the method of the first aspect comprises the steps of:
(i) providing aqueous compartments having a volume of more than 1 microlitre and comprising a single cell, and
(ii) splitting said compartments into microcompartments having a volume of up to 1 microlitre.

Aqueous compartments having a volume of more than 1 microlitre and comprising a single cell can be provided by using methods known in the art, e.g. as described in El Debs et al. (2012) Functional single-cell hybridoma screening using droplet-based microfluidics. Proc Natl Acad Sci U S A 109(29):11570-11575; Lecault et al. (2012), Microfluidic single cell analysis: from promise to practice, Current Opinion in Chemical Biology, Vol. 16, 3-4, August 2012, Pages 381-390; or Yin et al., (2012), Microfluidics for single cell analysis._Current Opinion in Biotechnology, 2012 Feb;23(1):110-9.

The compartments can be split in step (ii) by guiding them through outbranching microchannels and the compartments are split at the branching point. These outbranching microchannels can be channels that branch out into two or more channels, e.g. 2, 3, 4, 5 or more channels. When splitting the compartments, the flow rate in the branches can be controlled such that the compartments split into parts of certain volumes, e.g. 1:1 volume, 1:2 volume, 1:3 volume, 1:4 volume etc. The flow rate can be controlled, e.g., by the flow resistance of the channel or by suction. The compartments can be split once or more than once. In one embodiment, they (i.e. first they and then their daughter compartments) are split at least two (or at least 3, 4, 5, 6, 7, 8, 9 or 10) times at at least two (or at least 3, 4, 5, 6, 7, 8, 9 or 10) subsequent branching points.

In the methods of the first aspect, including embodiments A to D, each microcompartment may comprise at least two identifiers, preferably each comprising a constant sequence and a barcode sequence, wherein the constant sequence differs between the at least two identifiers and the barcode sequence differs or not between the at least two identifiers. In embodiments B and/or C of the method of the first aspect, each microcompartment may comprise these least two identifiers, wherein the at least two identifiers are added to each other using valves. In a preferred embodiment, these valves are controlled by a Braille display or they are multilayer soft lithography valves (see Thorsen T, Maerkl SJ, Quake SR. Science. 2002; 298 (5593): 580-4).

In a preferred embodiment of embodiments B and/or C of the method of the first aspect, the cell droplets are split into two or more smaller cell droplets to further reduce the size of the droplets. As the origin cell droplet comprises a single cell, only one of the resulting smaller droplets comprises a cell, while the others comprise no cell (nevertheless, they are termed cell droplets due to their origin).

Similarly, in the methods of the first aspect, including embodiments A to D, to further reduce the size of the microcompartments, the microcompartments are split into two or more smaller microcompartments. As each origin microcompartment comprises a single cell, only one of the resulting microcompartments comprises a cell, while the others comprise no cell.

In a preferred embodiment of the methods of the first aspect, the microcompartments have a volume of 0.1 to 500 nl. Preferably, the volume is less than 500 nanolitres, less than 250, less than 150, less than 100 or less than 50 nl. In a preferred embodiment, it is between 0,1 and 150 nl, preferably between 1 and 125 nl, between 10 and 100 nl or between 30 and 80 nl. In one preferred embodiment, it is about 70 nl.

In another embodiment of the methods of the first aspect, reagents to modify and/or amplify the cellular RNA or cellular DNA are added to the aqueous microcompartments. Preferably, such reagents are selected from the group consisting of reagents for a reverse transcription reaction, reagents for an *in vitro* transposition reaction, reagents which convert unmethylated cytosins and reagents for amplifying oligonucleotides, and combinations thereof; e.g. reagents for a reverse transcription reaction and reagents for an *in vitro* transposition reaction.

Reagents for a reverse transcription reaction preferably comprise a reverse transcriptase, aqueous buffers, salts and/or desoxynucleoside triphosphates. Suitable reagents are well known in the art, see, e.g., Molecular Cloning: A Laboratory Manual (4th Edition), Green & Sambrook 2012. Preferred are thermostable RNA polymerases, preferably in the presence of a RNA-dependent DNA polymerase including, without limitation, AMV, Cloned MMLV, SuperscriptII, ReverTraAce, Tth reverse transcriptase, hepatitis B reverse transcriptase, cauliflower mosaic virus reverse transcriptase, bacterial reverse transcriptase, Thermoscript. RSV reverse transcriptase, RAV (Rous-associated virus) reverse transcriptase, MAV (myeloblastosis-associated virus) reverse transcriptase, and HIV reverse transcriptase. See Kotewicz M, et al., US 5,244,797, and Gerard G, et al., WO1998047912. Preferably, they also comprise constant Smart-primers for the incorporation of specific 5' oligonucleotides during cDNA synthesis. These Smart-primers make use of a 3'-poly-C stretch unspecifically introduced into all newly synthesized cDNA molecules by some reverse transcriptases (e.g. the Moloney murine leukemia virus reverse transcriptase) during the first strand synthesis, based on its terminal transferase activity. The Smart primers consist of a sequence of interest (e.g. the T7 oligonucleotide sequence) plus a short poly-G stretch on their 3'-end hence allowing to partially hybridize with the cDNA molecules. When the RT reaches this point, it switches template and incorporates a sequence complementary to the remaining Smart primer sequence into the newly synthesized cDNA strand. Hence each cDNA molecule, independently of the gene it encodes, has defined sequences on both ends (poly-T and the complementary Smart primer sequence) and can thus be amplified by subsequent PCR.

Reagents for an *in vitro* transposition reaction preferably comprise a transposase, aqueous buffers and/or salts. Suitable reagents are well known in the art, see, e.g. Griffin et al., Nucl. Acids Res. (1999) 27 (19):3859-3865; Adey et al., Genome Biology 2010, 11:R119; Caruccio, Methods Mol Biol. 2011;733:241-55. A preferred transposase is the Tn5 transposase or the Mu transposase.

Reagents which convert unmethylated cytosins and reagents for amplifying oligonucleotides preferably comprise a reagent capable of converting an unmethylated cytosine to uracil or to another base that is detectably dissimilar to cytosine in terms of hybridization properties. The converting agent is preferably a bisulfite such as disulfite or hydrogen sulfite. The reaction is performed according to standard procedures (Frommer et al., 1992, Proc Natl Acad Sci USA 89:1827-31; Olek, 1996, Nucleic Acids Res 24:5064-6; EP 1394172). Reagents which amplify oligonucleotides, preferably ssequences comprising at least one CpG site preferably comprise a 3' primer capable of hybridizing to downstream of a specific CpG site, a DNA polymerase, aqueous buffers, salts and/or desoxynucleotide triphosphates. The 5' primer is provided by the identifier comprising a 3' sequence specific for bisulfit converted genomic DNA.

Reagents for amplifying oligonucleotides preferably comprise a polymerase, e.g. a DNA polymerase, aqueous buffers, salts, desoxynucleotide triphosphates and/or primers. Suitable reagents are well known in the art, see, e.g., Molecular Cloning: A Laboratory Manual (4th Edition), Green & Sambrook 2012.

If combined with reagents which convert unmethylated cytosines, it is preferred that the 3' sequence specific for bisulfit converted genomic DNA hybridizes to the genomic DNA upstream of sequence comprising at least one CpG site, i.e. the 3' sequence specific for bisulfit converted genomic DNA acts as a 5' primer. Optionally, a 3' primer which hybridises downstream of the sequence comprising at least one CpG site is added.

The polymerase used can be any polymerase known in the art to be appropriate for synthesizing a complementary nucleotide strand. In a preferred embodiment, the nucleotide polymerase is selected from the group consisting of mesophilic polymerases, thermophilic polymerases, DNA-dependent DNA polymerases if the oligonucelotides consist of DNA, RNA-dependent DNA polymerases if the oligonucelotides consist of RNA. Examples of polymerases are E.coli DNA polymerase, Klenow fragment DNA polymerase, Taq polymerase, Thermococcus kodakaraensis DNA polymerase, Thermococcus litoralis DNA polymerase, Pfu DNA polymerase, Pyrococcus DNA polymerase, Phusion DNA Polymerase, Thermus brockianus DNA polymerase, T4-DNA polymerase, T7-DNA polymerase, Bacillus stearothermophilus DNA polymerase, Sulfolobus DNA Polymerase IV, phi29 DNA Polymerase, M-MLV reverse transcriptase, HIV-1 reverse transcriptase, AMV reverse transcriptase, phi6 RNA polymerase or PyroPhage DNA Polymerase.

In these embodiments, it is preferred that the at least one identifier comprises a 3' oligo(dT) sequence (for a reverse transcription reaction), inverted repeats to make it a transposable element (for an *in vitro* transposition reaction), or a 3' sequence specific for bisulfit converted genomic DNA (for determining the methylation status).

The term "capable of hybridizing" or "capable of specifically hybridizing", as used herein, refers to the capacity of an oligonucleotide, polynucleotide or a sequence part thereof of recognizing a sequence specifically. As used herein, "hybridization" is the process of combining two complementary single-stranded nucleic acid molecules, or molecules with a high degree of similarity (at least in the sequence stretch that is capable of hybridising), and allowing them to form a single double-stranded molecule through base pairing. Normally, the hybridization occurs under highly stringent conditions or moderately stringent conditions.

As known in the art, the "similarity" between two nucleic acid molecules (at least in the sequence stretch that is capable of hybridising) is determined by comparing the nucleotide sequence of one molecule to the nucleotide sequence of a second molecule. Variants according to the present invention include nucleotide sequences that are at least 60%, 65%, 70%, 75%, 80%, 85%, 90%, or 95% similar or identical to the target sequence. Alternatively, they differ in up to 10, 9, 8, 7, 6, 5, 4, 3, 2, or 1 nucleotide from the target sequence. The degree of identity between two nucleic acid molecules is determined using computer algorithms and methods that are widely known for the persons skilled in the art. The identity between two amino acid sequences is preferably determined by using the BLASTN algorithm (BLAST Manual, Altschul et al., 1990, NCBI NLM NIH Bethesda, Md. 20894, Altschul, S., et al., J. Mol Biol 215:403-10).

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

The term "stringent conditions" or "high stringency conditions", as used herein, typically: (1) employ low ionic strength and high temperature for washing, for example 0.015 M sodium chloride/0.0015 M sodium citrate/0.1% sodium dodecyl sulfate at 50° C.; (2) employ during hybridization a denaturing agent, such as formamide, for example, 50% (v/v) formamide with 0.1% bovine serum albumin/0.1% Ficoll/0.1% polyvinylpyrrolidone/50 mM sodium phosphate buffer at pH 6.5 with 750 mM sodium chloride, 75 mM sodium citrate at 42°C.; or (3) employ 50% formamide, 5xSSC (0.75 M NaCl, 0.075 M sodium citrate), 50 mM sodium phosphate (pH 6.8), 0.1% sodium pyrophosphate, 5x Denhardt's solution, sonicated salmon sperm DNA (50 µg/ml), 0.1% SDS, and 10% dextran sulfate at 42°C, with washes at 42°C in 0.2xSSC (sodium chloride/sodium citrate) and 50% formamide, followed by a high-stringency wash consisting of 0.1xSSC containing EDTA at 55 °C.

"Moderately stringent conditions" may be identified as described by Sambrook et al., Molecular Cloning: A Laboratory Manual, New York: Cold Spring Harbor Press, 1989, and include the use of washing solution and hybridization conditions (e.g., temperature, ionic strength and % SDS) less stringent that those described above. An example of moderately stringent conditions is overnight incubation at 37°C. in a solution comprising: 20% formamide, 5xSSC (150 mM NaCl, 15 mM trisodium citrate), 50 mM sodium phosphate (pH 7.6), 5x Denhardt's solution, 10% dextran sulfate, and 20 mg/ml denatured sheared salmon sperm DNA, followed by washing the filters in 1xSSC at about 37-50°C. The skilled artisan will recognize how to adjust the temperature, ionic strength, etc. as necessary to accommodate factors such as probe length and the like.

In a second aspect, the present invention relates to an aqueous microcompartment having a volume of up to 1 microlitre, comprising:
- a single cell, and
- at least one identifier comprising a molecular barcode being unique for each microcompartment.

In a preferred embodiment, the identifier is a sequence identifier, preferably an oligonucleotide, comprising a barcode sequence and, preferably, a constant sequence, wherein the barcode sequence is unique for the microcompartment and the constant sequence may be identical to the constant sequence of sequence identifiers of other microcompartments.

In the most preferred embodiment, the microcompartment has been generated using any method of the first aspect of the invention.

All terms used to describe the second aspect of the invention have the meanings as defined for the first aspect of the invention. Also, all embodiments characterizing the aqueous microcompartments of the method of the first aspect also apply/characterize the aqueous microcompartment of the second aspect. For example, the microcompartment of the second aspect may have the volumes described as preferred above and may have the content as described above (e.g., the identifiers as described above).

In a third aspect, the present invention relates to a sample generated by pooling at least two aqueous microcompartments according to the second aspect of the invention.

The term "pooling", refers to pooling the microcompatment contents but not the microcompartments. In one embodiment, this can be achieved by fusing the microcompartments to be pooled. In another embodiment, it can be achieved by breaking microcompartments which have been collected in a common recipient. Collected microcompartments are broken by using any means known in the art, for example by addition of a destabilising agent, for example using an emulsion destabilizer such as perfluoro-octanol or A104 (RainDance Technologies) as described in Clausell-Tormos et al. (Droplet-based microfluidic platforms for the encapsulation and screening of mammalian cells and multicellular organisms, Chem Biol, vol 15, pg 427, 2008), or by the application of an electric field, or by freezing and thawing the emulsion, to release the combined identifiers into the aqueous phase. In yet another embodiment, this can be achieved by injecting the microcompartments into a reservoir (and optionally adding a emulsion destabilizer) where the phases separate due to their different densities. Then the upper aqueous phase is removed for further processing. Depending on whether or not the contents of the pooled microcompartments are still in a compartment/microcompartment or not, further reactions are either carried out in a microfluidics setting or in conventional bench-top setting.

In a fourth aspect, the present invention relates to a method for modifying, amplifying and/or analysing the DNA or RNA comprised in a single cell, wherein the cell is comprised in a microcompartment according to the second aspect for at least some of the method steps.

In an embodiment A, the method of the fourth aspect is a method for determining the RNA of a single cell, comprising the steps of
(i) providing an aqueous microcompartment according to the second aspect, wherein the at least one identifier comprises a 3' oligo(dT) sequence, and comprising reagents for a reverse transcription reaction,
(ii) lysing the single cell,
(iii) reverse transcribing mRNA released by the single cell to cDNA within the microcompartment,
(iv) amplifying the cDNA, and
(v) determining the presence and/or sequence of the cDNA.

Reagents for a reverse transcription reaction and for amplifying oligonucleotides are described above.

The single cell can be lysed by any suitable means which does not disrupt the integrity of the microcompartment. Suitable means are, for example, heat, cycles of freezing and thawing, laser irradition, sonication, mechanical lysis, e.g. shearing. In a preferred embodiment, the cells are lysed without using chemical lysis agents. Heat lysis is preferably performed by giving a short heat pulse, e.g. 55 to 75 °C for 5 to 60 s, more specifically about 65 °C for about 20 s.

In a microfluidics setting, heat lysis can be achieved via a flat metal plate mounted under a microfluidic chip, which is in thermal connection with the samples. By using glass slides embedded in the PDMS, or pre-soaking the chip in water, evaporation problems are ruled out. Alternatively, heat lysis can be achieved by using heating elements integrated into a microfluidic chip.

In step (iii), the mRNA is transcribed using methods known in the art, e.g. from Molecular Cloning: A Laboratory Manual (4th Edition), Green & Sambrook 2012.

In step (iv), the cDNA/DNA is amplified by methods known in the art, e.g. from Molecular Cloning: A Laboratory Manual (4th Edition), Green & Sambrook 2012. The cDNA may be amplified directly, e.g. using any PCR based method, or indirectly, e.g. by *in vitro* transcription (IVT) amplification (see, e.g., Hashimshony et al., Cell Reports, 2, 666-673, 2002), wherein the cDNA is transcribed again to obtain a plurality of mRNA molecules, which are then transcribed to cDNA again. Preferably, the template cDNA/DNA is amplified by a factor of 2⁵⁻³⁰, more preferably 2¹⁰⁻²⁵, which corresponds to about 5-30 or 10-15 PCR cycles. For indirect amplification methods such as IVT amplification, the incubation time and/or the amount or enzyme for transcription are adjusted accordingly. If not already comprised, reagents for amplifying an oligonucleotide (as defined herein) are added in the amplification step.

The sequence of the cDNA (and thereby the sequence of the corresponding mRNA) can be determined by any sequencing method known in the art (see, e.g. Molecular Cloning: A Laboratory Manual (4th Edition), Green & Sambrook 2012). Exemplary sequencing reactions include those based on techniques developed by Maxam and Gilbert (Proc. Natl. Acad Sci USA, 1977, 74:560) or Sanger (Sanger et al., 1977, Proc. Nat. Acad. Sci. USA, 74:5463). It is also contemplated that any of a variety of automated sequencing procedures may be utilized (Biotechniques (1995) 19:448), including sequencing by mass spectrometry. See, for example, U.S. Pat. No. 5,547,835 and international patent publication number WO 94/16101, U.S. Pat. No. 5,547,835 and international patent application number WO 94121822 and U.S. Pat. No. 5,605,798 and International Patent Application No. PCT/US96/03651; Cohen et al. (1996) Adv. Chromatogr. 36: 127-162; and Griffin et al. (1993) Appl Biochem Biotechnol 38:147-159. Yet other suitable sequencing methods are disclosed, for example, in U.S. Pat. No. 5,580,732 and U.S. Pat. No. 5,571,676.

In a preferred embodiment, it is determined by Next Generation Sequencing (NGS, also known as 2^{nd} or 3^{rd} generation sequencing). The concept behind NGS it that the bases of a small fragment of DNA are sequentially identified from signals emitted as each fragment is re-synthesized from a DNA template strand. NGS extends this process across millions of reactions in a massively parallel fashion, rather than being limited to a single or a few DNA fragments. This advance enables rapid sequencing of large stretches of DNA base pairs spanning entire genomes, with the latest instruments capable of producing hundreds of gigabases of data in a single sequencing run. See, e.g., Shendure and Ji, Nature Biotechnology 26, 1135-1145 (2008) or Mardis, Annu Rev Genomics Hum Genet. 2008;9:387-402. Suitably NGS platforms are available commercially, e.g. the Roche 454 platform, the Roche 454 Junior platform, the Illumina HiSeq or MiSeq platforms, or the Life Technologies SOLiD 5500 or Ion Torrent platforms.

In an embodiment B, the method of the fourth aspect is a method for sequencing DNA of a single cell, comprising the steps of
(i) providing an aqueous microcompartment according to the second aspect, wherein the at least one identifier comprises a transposable element, and comprising reagents for an *in vitro* transposition reaction,
(ii) lysing the single cell,
(iii) carrying out a transposition reaction within the microfluidic droplet,
(iv) sequencing the DNA.

Reagents for a transposition reaction are described above and the single cell can be lysed as descibed above.

Methods for carrying out a transposition reaction for subsequently sequencing the DNA, which are suitable for the microfluidics format, are known in the art, e.g. from Parkinson et al., Genome Res. 2012 January; 22(1): 125-133. The sequence of the DNA can be determined as described above by any sequencing method known in the art, preferably by Next Generation Sequencing.

In an embodiment C, the method of the fourth aspect is a method for determining the methylation status of the genomic DNA of a single cell, comprising the steps of
(i) providing an aqueous microcompartment according to the second aspect, wherein the at least one identifier comprises a 3' sequence specific for bisulfit converted genomic DNA, and comprising reagents for bisulfit conversion of unmethylated cytosine and an amplification reaction,
(ii) lysing the single cell,
(iii) carrying out a bisulfit conversion reaction and amplifying the bisulfit converted DNA within the micro compartment, and
(iv) determining the methylation status of at least one CpG position within the genomic DNA.

Reagents for the reactions of step (ii) and (iii) are described above and the single cell can be lysed as descibed above. In step (iii), it is preferred that the 3' sequence specific for bisulfit converted genomic DNA hybridizes to the genomic DNA upstream of sequence comprising at least one CpG site, i.e. the 3' sequence specific for bisulfit converted genomic DNA acts as a 5' primer. Optionally, a 3' primer hybridises downstream of the sequence comprising at least one CpG site. The methylation status of a CpG position is preferably determined by sequencing the amplified bisulfit converted DNA, wherein each amplicon preferably comprises at least one CpG site.

In an embodiment D, the method of the fourth aspect is a method for simultaneously sequencing RNA and DNA of the same single cell, comprising the methods of the embodiments A and B of the second aspect, wherein the aqueous microcompartment of step (i) comprises reagents for a reverse transcription reaction, reagents for an *in vitro* transposition reaction, and at least two identifiers as described in the first aspect, wherein at least one identifier comprises a 3' oligo(dT) sequence and at least one identifier comprises a transposable element.

In a preferred embodiment of the methods of the fourth aspect, including embodiments A to D, at least two aqueous microcompartments are processed in parallel until and including step (iii), and wherein the aqueous parts of the microfluidic droplets are pooled and processed together subsequent to step (iii).

In another preferred embodiment, the methods according to the fourth aspect, preferably those of embodiments A and D, the amplified cDNA or DNA is fragmented, preferably to fragments having a length of 50 to 500 bp, more preferably about 200 bp. The fragmentation may lead to some fragments without a sequence identifier or barcode sequence, however, the sequence identifier at the 3' end is sufficient for trancsriptome analyses, as the 3' end is suffiently large to determine which gene sequence was expressed.

In yet another preferred embodiment, the cDNA or DNA (in particular the fragmented cDNA or DNA) is end-repaired and/or is blunt-end ligated to a 5' sequencing primer and/or a 3' sequencing primer. Sequencing primers for NGS sequencing are preferred, e.g. those of the NGS platforms mentioned above (for example Illumina sequencing primers are used). More preferably, the cDNA or DNA for sequencing is immobilised prior to sequencing, preferably on beads. Even more preferably, the immobilised cDNA or DNA is washed. Optionally, the cDNA or DNA (amplicons or fragments) are selected for sequencing by size.

In a preferred embodiment, the cDNA or DNA is fragmented using a microfluidic sonicator based on a Langevin-type piezo transducer coupled to a microfluidic channel network via the flexural lamb wave mode. This setup allows shearing of DNA, as well as covalently cross-linked chromatin, into fragments ranging from 180 bp to 4 kb. The size range can be controlled by the applied power and the duration of the sonication.

Furthermore, the methods of the fourth aspect allow to perform digital PCR in subsequent steps. In a preferred embodiment of these methods, the pooled sample can be emulsified into microfluidic droplets, wherein these droplets have a statistical likelihood of less than 1 of containing one cDNA/DNA template. The amplification step of the methods of the fourth aspect is carried out in these droplets and the droplets are then pooled for analysis by, e.g. sequencing. Thereby, amplification artefacts can be almost completely excluded.

As a most preferred embodiment of the methods of the fourth aspect, including embodiments A to D, the steps prior to sequencing or determining the methylation status are carried out in a microfluidics system, preferably comprising one or more of the following:
- a microfluidic chip,
- a microfluidic bead column,
- heating means for a micro fluidic chip,
- a microfluidic sonicator.

In a fifth aspect, the present invention relates to an apparatus and to a method for producing a microfluidic or aqueous microcompartment containing a single cell. More specifically, it relates to an apparatus for producing a microfluidic or aqueous microcompartment containing a single cell, comprising
- an aspirator, e.g. a needle, for aspirating a microfluidic droplet from the aqueous phase of a two-phase liquid, and
- a control means for adjusting the depth of immersion of the aspirator in the two-phase liquid, which ensures that the aspirator aspirates the cell from the aqueous phase at the interface of the two-phase liquid.

Also, it relates to a method for producing a microfluidic or aqueous microcompartment comprising a single cell, comprising the steps of:
(i) immersing an aspirator in the water phase of a two-phase liquid at the interface of the two phases, wherein the aqueous phase comprises a single cell sedimented at the interface,
(ii) aspirating the cell from the aqueous phase at the interface of the two phases.

In a preferred embodiment, the non-aequous phase is an oil as defined above. The cell is aspirated in a volume which is equivalent to that of the microcompartment, which is as defined above.

### EXAMPLES

### Example 1: Microfluidic RNAseq Platform

The inventor has optimized the microfluidics technology for high-throughput single-cell/single-virus assays and biochemical screens (e.g. to screen antibody-, peptide- and small-molecule based enzyme inhibitors), see e.g. Fig. 1. Furthermore, they have developed continuous flow systems for next generation sequencing (NGS) applications, e.g. for the fragmentation of DNA using sub-microliter sample volumes. The present invention relates, inter alia, to high-throughput, high-efficiency single cell RNAseq. The complete workflow is shown in Fig. 2: Individual cells are seeded into microtiter plates (optionally in an automated fashion through a prior FACS sort, which also allows selecting particular cells from a heterogeneous population) and all reagents for the reverse transcription (RT) step are added (e.g. using liquid handling robots).

Poly-T primers harbouring a unique barcode for each sample and constant Smart-primers for the incorporation of specific 5'-oligonucleotides during cDNA synthesis (based on the template switching and terminal transferase activity of Moloney murine leukemia virus RT (Ramskold D, et al. (2012) Full-length mRNA-Seq from single-cell levels of RNA and individual circulating tumor cells. Nature Biotechnology 30(8):777-782)) are provided in each well. In the next step, an advanced and newly developed version of a custom-build two-phase autosampler (see Fig. 3 and Example 2 for details) is used to load these samples from the microtiter plates into a target tubing in form of aqueous 70 nL plugs spaced out by immiscible oil. The samples are then transferred to a microfluidic chip with an embedded heating element (see Fig. 5D and Example 4 for details) to initiate cell lysis by a short heat pulse (65 °C for 20 sec; note that this does not irreversibly inactivate the RT present in each sample). Subsequently, the temperature is adjusted to 42°C to allow for efficient cDNA synthesis. After an incubation time of about 90 min, the samples are recovered and pooled by extraction of the liquid phase (Fig. 4A). At this stage, all cDNAs synthesized from a particular cell have the same barcode, hence preserving single cell data. Furthermore, by knowing which barcode was present in which well of the initial microtiter plate, the later sequencing results can clearly be assigned to particular cells. This is of special interest in case the single cells were not "randomly" seeded by FACS, but rather obtained by imaging-based approaches (e.g. picking individual cells from a heterogeneous sample using a micro-manipulator).

In the next step, the pooled cDNA sample is amplified by 10-15 cycles of PCR. For this purpose, PCR reagents are added to the liquid phase extract (if required prior on- or off-chip washing and purification steps can be performed; see Fig. 4C and Example 6 for details) before it is reinjected into the microfluidic PCR device, optionally using emulsion PCR (ePCR) to minimize amplification artefacts (see Fig. 5 and Example 5 for details). Subsequently, the sample is flushed into a sonication chamber for DNA fragmentation. Using their own microfluidic sonicator, the inventor can easily generate fragments of about 200 bp with narrow peak width (Fig 4B), exactly as required for NGS. In further step reagents for DNA end repair are added and adaptors (e.g. harbouring Illumina primer sequences) are blunt-end ligated to the fragments. Ultimately, size selection is performed using microfluidic columns filled with Ampure XP beads. After sufficient washing steps the sample is eluted and transferred (by simple pipetting from the outlet - this step has also been demonstrated for the microfluidic sonicator of the inventor) to the sequencing machine.

Especially for performing the reverse transcription and the pre-amplification step in tiny sample volumes, the system of the invention has massively improved efficiencies and hence decreased sequencing biases.

Furthermore, the inventor combined automated set up of single cell samples with on-chip reverse transcription (including the introduction of unique barcodes for each sample; first 2 steps in Fig. 2). As the samples can be pooled afterwards, only a single sample has to be manipulated further downstream. This not only increases the throughput massively compared to current manual protocols, but as well allows using much bigger volumes in the subsequent steps. Hence loss of material can be minimized (when transferring samples from microfluidic chips to bench top instruments), resulting in improved sensitivity. Furthermore, this approach also enables to perform digital PCR in the subsequent steps: The pooled sample can be emulsified into millions of water-in-oil droplets (with on average less than one cDNA template per droplet) thus excluding amplification artefacts almost completely (see Example 4 for details).

A further application of the combined single-cell sample preparation and barcoding platform is DNA/genome sequencing: The RT step is replaced with an *in vitro* transposition reaction in which barcodes are introduced across the entire genome (which, at the same time is fragmented), the envisaged system can easily be used for this task. *In vitro* transposition-based barcoding systems are commercially available (e.g. Nextera; www.epibio.com/nextera) and transforming them into a droplet-based format allows performing the most time- and labour-intense steps of single cell genome sequencing in an automated way, hence allowing drastically improved throughput and reliability. Furthermore, simultaneous DNA and RNA sequencing can be combined when combining RT and *in vitro* transposition and using poly-T- and transposon-specific primers for the two different reactions.

### Example 2: Module for the automated generation of single-cell plugs

The inventor developed an automated system for the loading of samples from microtiter plates into a target tubing, where they are stored in form of aqueous plugs spaced out by immiscible oil (Fig. 3). These samples can be easily transferred onto microfluidic chips for further processing. While the initial system was based on a modified, commercial autosampler (Dionex 3000 SL series) and had certain limitations in terms of minimum sample size (1-5µl) and throughput (> 30s/sample), the inventor now developed a custom-build second generation version of this platform. This new instrument not only enables them to generate plugs with a volume of no more than 70 nL at a rate of about 5s/sample, but as well facilitates the handling of cells: When seeding the cells individually into the wells of a microtiter plate (e.g. based on a prior FACS sort), the system can generate chemically distinct sample plugs, each one hosting exactly one cell (see inset of Fig. 4). This is achieved by pre-filling the wells with dense fluorinated oil and adjusting how much the needle of the autosampler is immersed in the (two-phase) well: As the cell is sedimenting to the interface it is always included in the resulting plug, even when using minute sample volumes.

### Example 3: Extraction Module

After having generated and manipulated plugs hosting individual cells, the aqueous samples are recovered and pooled using an extraction module (Fig. 4A), working similar to a liquid exchanger: The plugs are injected into a large reservoir where the phases separate due to their different densities. Then the upper aqueous phase can be easily removed for further processing. For example, it can be transferred to another microftuidic chip for on-chip PCR.

### Example 4: Heating/PCR Module

Many microfluidic chips for enzymatic reactions and PCR have been developed over the last years. The inventor developed two complementary approaches in their lab (Fig. 5A-C): On one hand they have adapted a commercial PCR device for thermocycling of entire microfluidic chips, by adding a flat metal plate (on which the microfluidic chip can be mounted), which is in thermal connection with the sample racks and hence cycles through the temperatures. Using glass slides embedded in the PDMS, or pre-soaking the chip in water, evaporation problems are ruled out. In an alternative approach, the inventor has directly integrated heating elements into the microftuidic chip (Fig. 5D): By measuring the temperature using embedded thermistors and adjusting the applied voltages, feedback loops for efficient cycling can be generated.

As a further option, aqueous samples can also be emulsified into surfactant-stabilized droplets to perform digital PCR: By diluting the sample in a way that, on average, less than one template molecule ends up in each droplet, biases due to PCR recombination artefacts or the competition of different (e.g. long and short; high and low abundant) templates for reagents (polymerase, primers, nucleotides) can be ruled out. This approach can also be performed with the two above systems. Again, after completion of the PCR reaction, the aqueous samples can be recovered and pooled using an extractor (Fig. 4A) and adding an emulsion breaker such as perfluoro-octanol.

### Example 5: Microfluidic Sonicator

Once a cDNA library has been amplified, the sample can be sheared in the microfluidic sonicator of the inventor (Fig. 4B): This device is based on a Langevin-type piezo transducer coupled to a microftuidic channel network via the flexural lamb wave mode. This setup allows shearing of DNA, as well as covalently cross-linked chromatin, into fragments ranging from 180 bp to 4 kb. The size range can be easily controlled by the applied power and the obtained size distribution is comparable to that of macroscopic benchtop devices. Downstream immunoprecipitation experiments even demonstrated that the processed material can be easily recovered for further off-chip manipulation steps.

### Example 6: Sample Washing and Purification Modules

After shearing of the pre-amplified samples, the DNA is end repaired and sequencing primers are introduced by blunt-end ligation. For these steps, the newly synthesized cDNA library is, advantageously, immobilized (e.g. on Ampure XP beads) and washed. For this purpose, the inventor developed microfluidic bead columns and magnetic bead traps (Fig. 4C): Samples such as DNA can be captured on functionalized beads immobilized inside a microfluidic channel. Hence they can be incubated sequentially with different reagents (e.g. enzymes), washed and eluted. Alternatively even the beads themselves can be transported to the next reaction chamber on a microfluidic chip when using magnetic control.

## Claims

1. A method for generating a plurality of aqueous microcompartments having a volume of up to 1 microlitre, each comprising:
- a single cell, and
- at least one identifier comprising a molecular barcode being unique for each microcompartment,
wherein the method comprises the steps of
(A):
(i) providing an array of wells,
(ii) seeding cells comprised in an aqueous medium into at least two wells of the array of wells such that only one cell is seeded into each well,
(iii) adding at least one identifier into at least two wells of the array of wells, such that each of the at least two wells is assigned a different barcode sequence, and
(iv) adding to at least two wells a liquid, which is immiscible with the aqueous medium comprising the cells of (ii) and has a higher density than the aqueous medium,
(v) in an alternating fashion, (a) aspirating the volume of up to 1 microlitre at the interphase after the cell has sedimented at the interphase from one well into a microchannel containing the immiscible liquid, and (b) filling the microchannel with the immiscible liquid behind the aspirated aequous volume,
wherein steps (ii), (iii) and (iv) can be carried out in any order or simultaneously;
or (B):
(i) injecting (a) an aqueous medium comprising cells and (b) sequentially at least one identifier per cell into a microchannel to create a laminar co-flow of cells and identifiers, wherein the sequential injection of the identifiers is controlled by at least one valve"
(ii) generating cell droplets in the microchannel using microfluidic flow focusing from the laminar co-flow of step (i), wherein the concentration of the cells in the medium and the droplet size are adjusted such that each drop has a statistical likelihood of comprising less than one cell;
or (C):
(i) generating cell droplets in a first microchannel using microfluidic flow focusing from an aqueous medium comprising cells, wherein the concentration of the cells in the medium and the drop size are adjusted such that each drop has a statistical likelihood of comprising less than one cell,
(ii) generating oligonucleotide droplets in a second microchannel, preferably using microfluidic flow focusing, from a liquid comprising the at least one identifier, wherein each droplet comprises an identifier with a different barcode sequence,
(iii) fusing each cell droplet with an identifier droplet,
wherein steps (i) and (ii) can be carried out in any order or simultaneously;
or (D):
(i) providing aqueous compartments having a volume of more than 1 microlitre and comprising a single cell, and
(ii) splitting said compartments into microcompartments having a volume of up to 1 microlitre.

2. The method of claim 1, wherein in step (B) (i), the sequential injection of the identifiers is controlled either by one valve for all identifiers, fed with one identifier after the other, or by one valve per identifier, wherein one valve opens after the other and not more than one valve is open at any time.

3. The method of any one of claims 1 to 2, wherein the at least one identifier is a sequence identifier comprising a constant sequence and a barcode sequence, wherein the constant sequence is identical for all microcompartments and the barcode sequence is unique for each microcompartment.

4. The method of claim 3, wherein each microcompartment comprises at least two identifiers, each comprising a constant sequence and a barcode sequence, wherein the constant sequence differs between the at least two identifiers and the barcode sequence differs or not between the at least two identifiers.

5. The method of any one of claims 1 to 4, wherein the cell droplets and/or the microcompartments are split into two or more smaller droplets or microcompartments, respectively.

6. The method of any one of claims 1 to 5, wherein reagents to modify and/or amplify the cellular RNA or cellular DNA are added to the aqueous microcompartments.

7. The method of claim 6, wherein the reagents are selected from the group consisting of reagents for a reverse transcription reaction; reagents for an *in vitro* transposition reaction; reagents which convert unmethylated cytosins and reagents for amplifying oligonucleotides; and combinations thereof.

8. The method of any one of claims 1 to 7, wherein the at least one identifier comprises a 3' oligo(dT) sequence, a transposable element for an *in vitro* transposition reaction or a 3' sequence specific for bisulfit converted genomic DNA.

9. The method of any one of claims 1 to 8, wherein the microcompartments have a volume of about 70 nl.

10. An aqueous microcompartment having a volume of up to 1 micro litre, comprising:
- a single cell, and
- at least one identifier comprising a molecular barcode being unique for each microcompartment.

11. The aqueous microcompartment of claim 10, generated with the method of any one of claims 1 to 8.

12. A sample generated by pooling at least two aqueous microcompartments of claim 10 or 11.

13. A method for modifying, amplifying and/or analysing the DNA or RNA comprised in a single cell, wherein the cell is comprised in a microcompartment according to claim 10 or 11 for at least some of the method steps.

14. The method of claim 13, wherein the method is selected from the group consisting of:
(A): a method for determining the RNA of a single cell, comprising the steps of
(i) providing an aqueous microcompartment according to claim 10 or 11, wherein the at least one identifier comprises a 3' oligo(dT) sequence, and comprising reagents for a reverse transcription reaction,
(ii) lysing the single cell,
(iii) reverse transcribing mRNA released by the single cell to cDNA within the microcompartment,
(iv) amplifying the cDNA, and
(v) determining the presence and/or sequence of the cDNA;
or (B): a method for sequencing DNA of a single cell, comprising the steps of
(i) providing an aqueous microcompartment according to claim 10 or 11, wherein the at least one identifier comprises a transposable element, and comprising reagents for an *in vitro* transposition reaction,
(ii) lysing the single cell,
(iii) carrying out a transposition reaction within the microfluidic droplet,
(iv) sequencing the DNA;
or (C): a method for determining the methylation status of the genomic DNA of a single cell, comprising the steps of
(i) providing an aqueous microcompartment according to the claim 10 or 1 l, wherein the at least one identifier comprises a 3' sequence specific for bisulfit converted genomic DNA, and comprising reagents for bisulfit conversion of unmethylated cytosine and an amplification reaction,
(ii) lysing the single cell,
(iii) carrying out a bisulfit conversion reaction and amplifying the bisulfit converted DNA within the microcompartment, and
(iv) determining the methylation status of at least one CpG position within the genomic DNA;
or (D): a method for simultaneously sequencing RNA and DNA of the same single cell, comprising the methods of (A) and (B), wherein the aqueous microcompartment of step (i) comprises reagents for a reverse transcription reaction, reagents for an *in vitro* transposition reaction, and at least two identifiers, wherein at least one identifier comprises a 3' oligo dT sequence and at least one identifier comprises a transposable element.

15. The method of claim 13, wherein at least two aqueous microcompartments are processed in parallel until and including step (iii), and wherein the aqueous parts of the microfluidic droplets are pooled and processed together subsequent to step (iii).
